# EUROPEAN PATENT APPLICATION

(11) **EP 3 701 918 A1**
(43) Date of publication of application: **02.09.2020**
(21) Application number: 18870060.3
(22) Date of filing: 04.07.2018
(51) Int. Cl.: A61F 9/007

(54) **IMPLANT AND IMPLANT SYSTEM**

(30) Priority: 23.10.2017 JP 2017204464
(71) Applicant: Doxnet Inc., Kyoto-shi, Kyoto 600-8443 (JP)
(72) Inventor: OTAKA Isao, Yokohama-shi Kanagawa 220-0004 (JP)
(74) Representative: Plougmann Vingtoft a/s
(86) International application number: PCT/JP2018/025378
(87) International publication number: WO 2019/082448

(57) **Abstract**

The implant (10) is attachable to an eye (200). The implant (10) includes a tube section (1) and a throttle section (2). A flow path (11) for aqueous humor lies inside the tube section (1). The throttle section (2) is arranged in the flow path (11). The tube section (1) has a first opening section (12) and a second opening section (13). The first opening section (12) allows communication between the flow path (11) and the outside (1c) of the tube section (1). The second opening section (13) allows communication between the flow path (11) and the outside (1c) of the tube section (1). The throttle section (2) includes a wall section (21) arranged in the flow path (11) and a bore section (22) penetrating the wall section (21).

## Description

### [TECHNICAL FIELD]

The present invention relates to an implant and an implant system.

### [BACKGROUND ART]

The implant described in Patent Literature 1 includes a plate and a discharge tube. The plate is sewn to the sclera. The discharge tube is arranged between the plate and the anterior chamber. When the implant has been attached to the eye, aqueous humor in the anterior chamber is discharged to the plate via the discharge tube. As a result, it is possible to prevent an abnormal rise of ocular pressure.

### [CITATION LIST]

### [Patent Literature]

[Patent Literature 1]
Japanese Translation of PCT International Application Publication No. H10-503405

### [SUMMARY OF INVENTION]

### [Technical Problem]

However, when the amount of aqueous humor discharged to the plate is too little, the aqueous humor is not sufficiently discharged from the anterior chamber to the outside of the eye. It is therefore difficult to lower the ocular pressure to a target level.

Typically, to attach an implant to the eye, the discharge tube is bound by using a tightening thread. Further, the amount of aqueous humor discharged by the implant is regulated according to the strength of tightening force of the tightening thread used for tightening the discharge tube. More specifically, the stronger the tightening force is, the smaller is the discharge amount of aqueous humor by the implant. Further, after an implant is attached to the eye, there are some situations where the amount of aqueous humor discharged by the implant decreases, and it is not possible to lower the ocular pressure to a desired level. In those situations, a practitioner cuts the tightening thread using laser light. When the tightening thread is cut, the discharge tube is released from the tightening force of the tightening thread that has been cut, so that the amount of aqueous humor discharged by the implant increases. As a result, the ocular pressure is lowered.

However, in cutting the tightening thread, it has been difficult for the practitioner to recognize the strength of the tightening force of the tightening thread to be cut. For this reason, it has been difficult for the practitioner to predict how much the amount of aqueous humor discharged by the implant will be increased and how much the ocular pressure will be lowered as a result of cutting the tightening thread. Consequently, practitioners cut the tightening thread without predicting the degree by which the ocular pressure will be lowered. It has therefore been difficult to smoothly perform the procedure of adjusting the ocular pressure.

In view of the problems described above, it is an object of the present invention to provide an implant and an implant system that make it possible to smoothly perform the procedure of adjusting the ocular pressure after the implant is attached to the eye.

### [Solution to Problem]

An implant disclosed in the present application is attachable to an eye. The implant includes a tube section and a throttle section. A flow path for aqueous humor lies inside the tube section. The throttle section is arranged in the flow path. The tube section has a first opening section and a second opening section. The first opening section allows communication between the flow path and the outside of the tube section. The second opening section allows communication between the flow path and the outside of the tube section. Each of the plurality of throttle sections includes a wall section arranged in the flow path and a bore section penetrating the wall section.

It is preferable to configure the implant disclosed in the present application so that the wall section is removable from the flow path.

It is preferable to configure the implant disclosed in the present application so that the wall section is removed from the flow path by being irradiated with laser light.

It is preferable to configure the implant disclosed in the present application so that the tube section passes the laser light, and the wall section absorbs the laser light.

It is preferable to configure the implant disclosed in the present application so that the tube section has one of a transparent color and a translucent color, and the wall section has a color that is visible via the tube section.

It is preferable to configure the implant disclosed in the present application so that the tube section includes a prescribed part that is positioned between the sclera of the eye and the conjunctiva of the eye while the implant is attached to the eye. It is preferable that the flow path includes a prescribed flow path positioned inside the prescribed part of the tube section. It is preferable that the throttle section is positioned in the prescribed flow path.

It is preferable to configure the implant disclosed in the present application so that, while the implant is attached to the eye, each of the plurality of wall sections is removed from the flow path by being irradiated with one of: the laser light that passes through the conjunctiva of the eye, the Tenon capsule of the eye, and the prescribed part of the tube section; and the laser light that passes through the conjunctiva of the eye and the prescribed part of the tube section.

It is preferable to configure the implant disclosed in the present application so that the wall section is slanted with respect to a perpendicular direction that is a direction perpendicular to a flow path direction. It is preferable that the flow path direction is a direction extending from the first opening section toward the second opening section via the flow path.

It is preferable to configure the implant disclosed in the present application so that a plurality of the throttle sections are arranged in the flow path. It is preferable that the plurality of throttle sections are arranged in the flow path direction. It is preferable that the flow path direction is a direction extending from the first opening section toward the second opening section via the flow path. It is preferable that the plurality of throttle sections are configured in such a manner that the more downstream the throttle section is positioned in terms of the flow path direction, the larger an area of the bore section is.

An implant system disclosed in the present application includes the above described implant, an irradiating section, and a measuring section. The irradiation section irradiates the laser light. The measuring section measures ocular pressure.

### [Advantageous Effects of Invention]

According to the present invention, it is possible to smoothly perform the procedure of adjusting the ocular pressure after the implant is attached to the eye.

### [BRIEF DESCRIPTION OF DRAWINGS]

[FIG. 1]

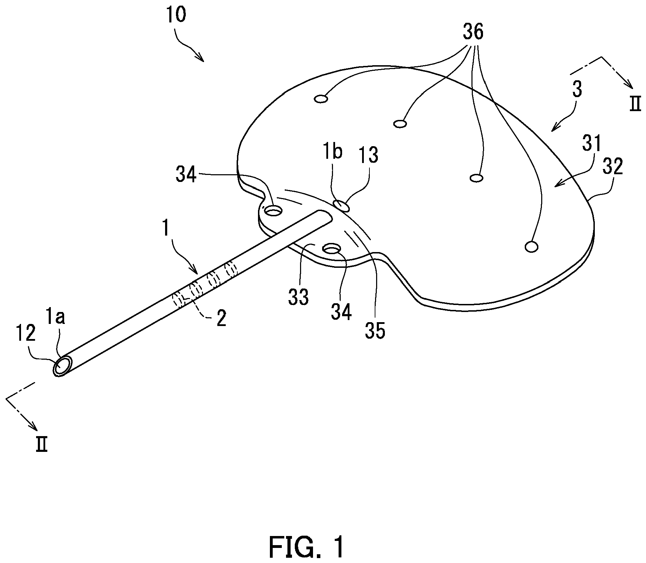

   FIG. 1 is a perspective view of an implant according to an embodiment of the present invention.
[FIG. 2]

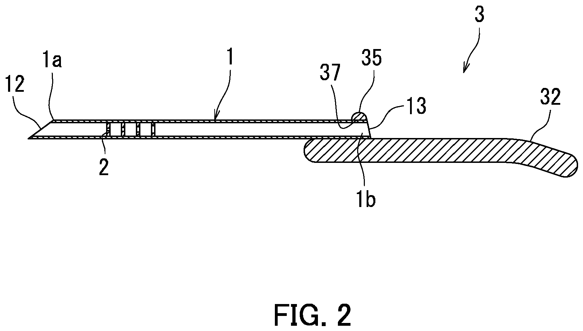

   FIG. 2 is a cross-sectional view taken at II-II in FIG 1.
[FIGS. 3A and 3B]

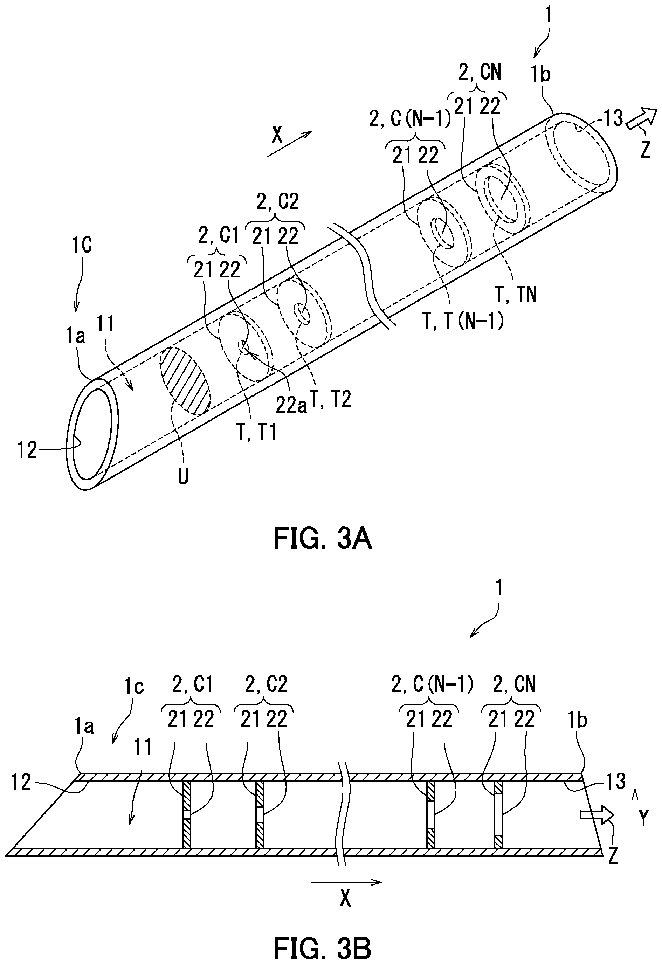

   FIG. 3A is a perspective view of a tube section. FIG. 3B is a cross-sectional view of the tube section.
[FIG. 4]

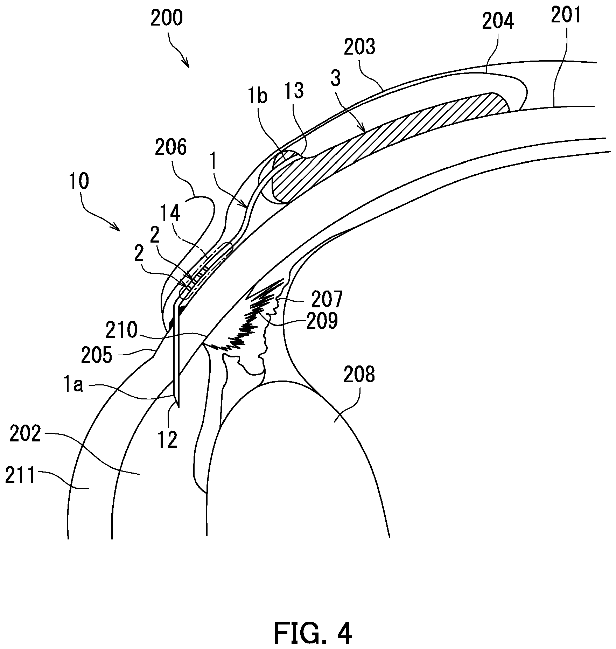

   FIG. 4 is a schematic cross-sectional view of an implant attached to an eye.
[FIG. 5]

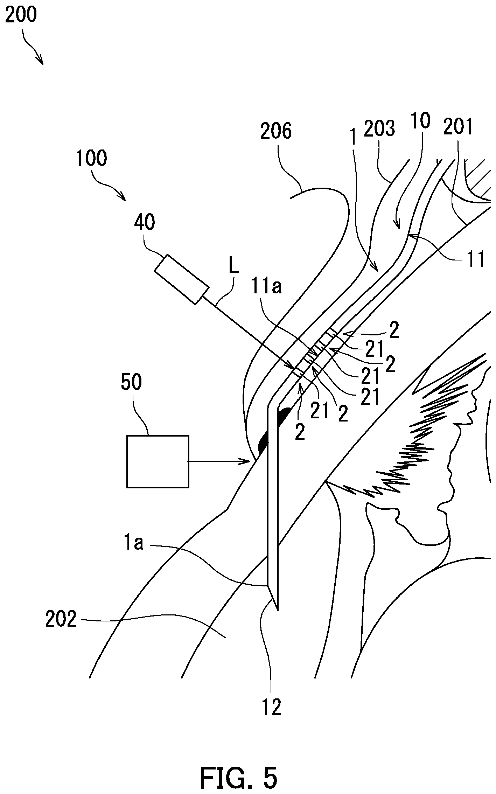

   FIG. 5 is a partial enlarged view of FIG. 4.
[FIG. 6]

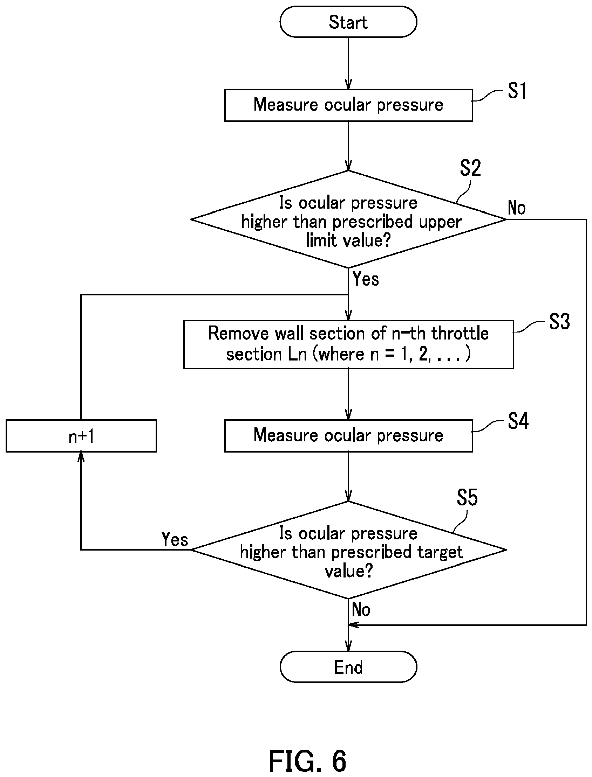

   FIG. 6 is a flowchart illustrating a procedure of adjusting the ocular pressure of the eye to which the implant has been attached.
[FIG. 7]

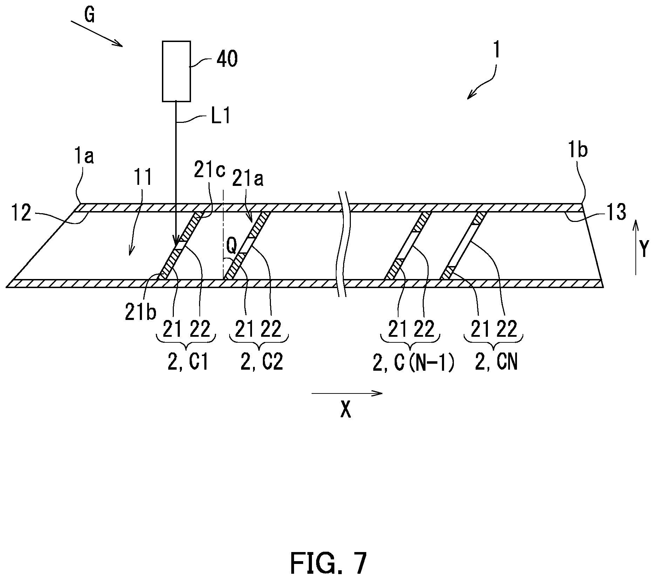

   FIG. 7 is a cross-sectional view illustrating a variation of wall sections.
[FIG. 8]

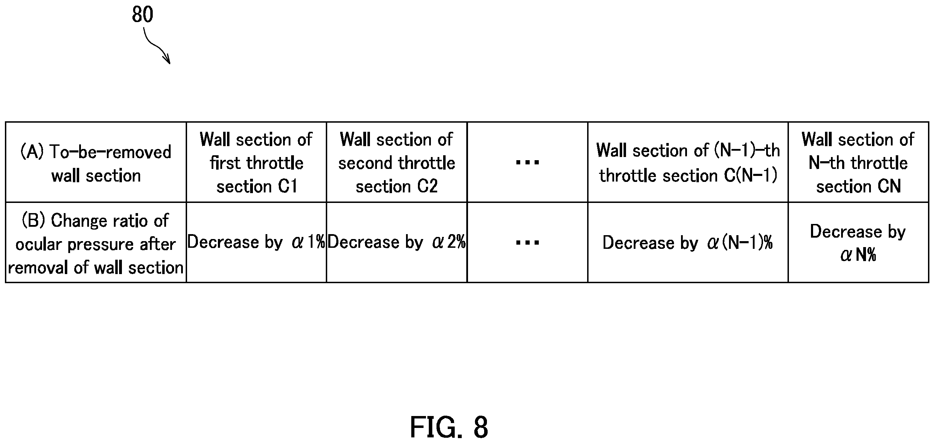

   FIG. 8 is a diagram illustrating change information.

### [DESCRIPTION OF EMBODIMENTS]

Embodiments of the present invention will be described with reference to the drawings. Some of the constituent elements in the drawings that are the same as or correspond to each other will be referred to by using the same reference characters, and the description thereof will not be repeated.

An implant 10, which is an embodiment of the present invention, will be described, with reference to FIGS. 1 and 2. FIG. 1 is a perspective view of the implant 10. FIG. 2 is a cross-sectional view taken at II-II in FIG. 1.

As illustrated in FIGS. 1 and 2, the implant 10 is used for treating glaucoma. The implant 10 is attachable to the eye. When the implant 10 has been attached to the eye, the implant 10 promotes aqueous humor to flow out of the eye. As a result, it is possible to prevent an abnormal rise of the ocular pressure.

The implant 10 includes a tube section 1, throttle sections 2, and a plate section 3.

The tube section 1 has a tubular shape. In other words, the tube section 1 is shaped to be hollow. The tube section 1 has one or more of flexibility, pliability, and elasticity. The tube section 1 is formed of, for example, one of the following: silicone; elastomer resin; and a resin material having elasticity. The tube section 1 in the present embodiment is a tube made of silicone.

The tube section 1 includes a first end section 1a and a second end section 1b. The tube section 1 has an oblong shape extending from the first end section 1a toward the second end section 1b.

The throttle sections 2 are arranged inside the tube section 1.

The plate section 3 has a plate-like shape. For example, the plate section 3 has one of the following, in a plan view: a circular shape, an oval shape, and a rectangular shape of which the corners are rounded.

The plate section 3 includes a main surface 31 and a main body section 32. The main surface 31 of the plate section 3 is located on the main body section 32. The area of the main surface 31 of the plate section 3 is, for example, in the range of 100 square millimeters to 600 square millimeters, inclusive. The main surface 31 of the plate section 3 is shaped to have a plane curved along the surface of the eyeball. The curvature radius of the main surface 31 of the plate section 3 is, for example, in the range of 12 millimeters to 14 millimeters, inclusive. The thickness of the plate section 3 is, for example, in the range of 0.5 millimeters to 2 millimeters, inclusive.

For example, the plate section 3 is formed of a material having one of: flexibility, pliability, and elasticity. More specifically, the plate section 3 is formed of, for example, a material containing a silicone elastomer. As a result, it is possible to change the shape of the plate section 3 so as to have the plane curved along the surface of the eyeball during the implant 10 being attached to the eye.

To the plate section 3, the tube section 1 is fixed. More specifically, to the plate section 3, the second end section 1b of the tube section 1 is fixed. The second end section 1b of the tube section 1 is positioned on top of the plate section 3 (the main body section 32). In contrast, the first end section 1a of the tube section 1 is positioned outside the plate section 3.

The plate section 3 further includes a bulging section 33, suture holes 34, a ridge section 35, a plurality of through holes 36, and an insertion hole 37. The bulging section 33 bulges out from the main body section 32. The suture holes 34 are located in the bulging section 33. The suture holes 34 penetrate the bulging section 33.

The ridge section 35 is shaped to project upward from the main body section 32. The insertion hole 37 is located between the ridge section 35 and the main body section 32. The tube section 1 is inserted in the insertion hole 37. While in the state of being inserted in the insertion hole 37, the tube section 1 is fixed to the main body section 32. More specifically, the second end section 1b of the tube section 1 is fixed to the main body section 32 while in the state of being inserted in the insertion hole 37.

Each of the plurality of through holes 36 penetrates the plate section 3. More specifically, each of the plurality of through holes 36 penetrates the main body section 32 of the plate section 3.

Next, the tube section 1 and the throttle sections 2 will be described, with reference to FIGS. 3A and 3B. FIG. 3A is a perspective view of the tube section 1. FIG. 3B is a cross-sectional view of the tube section 1.

As illustrated in FIGS. 3A and 3B, a flow path 11 for the aqueous humor extends inside the tube section 1. The flow path 11 denotes the space enclosed by the inner surface of the tube section 1. The tube section 1 guides the aqueous humor generated by the eye to the outside of the eye through the flow path 11. Further, the tube section 1 has a first opening section 12 and a second opening section 13 located therein. The respective opposite end sections of the tube section 1 are opened by a first opening section 12 and a second opening section 13. The first opening section 12 is located in the first end section 1a. The first opening section 12 allows communication between the flow path 11 and the outside 1c of the tube section 1. The second opening section 13 is located in the second end section 1b. The second opening section 13 allows communication between the flow path 11 and the outside 1c of the tube section 1. The tube section 1 discharges the aqueous humor that has flowed into the flow path 11 via the first opening section 12, out of the flow path 11 via the second opening section 13.

The throttle sections 2 are arranged in the flow path 11. The throttle sections 2 regulate the amount of aqueous humor to be discharged from the flow path 11 (the second opening section 13) per unit time period, by reducing a flow path area U of the flow path 11. The flow path area U denotes the cross-sectional area of the flow path 11 observed when the flow path 11 is sectioned in a direction perpendicular to a flow path direction X. Further, the flow path direction X denotes the direction extending from the first opening section 12 toward the second opening section 13 via the flow path 11. In other words, the flow path direction X denotes the extending direction of the tube section 1.

Each of the throttle sections 2 includes a wall section 21 and a bore section 22. The wall section 21 has a plate-like shape. The wall section 21 is arranged in the flow path 11. In other words, in the flow path 11, the wall section 21 prohibits communication between the upstream side of the wall section 21 and the downstream side of the wall section 21. In this situation, the upstream side of the wall section 21 denotes, more specifically, the upstream side of the wall section 21 in terms of the flow path direction X. The downstream side of the wall section 21 denotes, more specifically, the downstream side of the wall section 21 in terms of the flow path direction X.

The wall section 21 is formed of, for example, the same material as that forming the tube section 1. Alternatively, the wall section 21 may be formed of a material different from the material forming the tube section 1. The wall section 21 may be fixed to the flow path 11 or may be integrated with the flow path 11. In other words, the wall section 21 may be integral to the tube section 1 or may be separate from the tube section 1.

The wall section 21 has the bore section 22 formed therein. The bore section 22 penetrates the wall section 21. More specifically, the bore section 22 penetrates the wall section 21 in the flow path direction X. Accordingly, in the flow path 11, the upstream side of the wall section 21 and the downstream side of the wall section 21 communicate with each other via the bore section 22. In other words, in the location where the wall section 21 is positioned, the bore section 22 forms the flow path 11. Possible examples of the bore sections 22 include not only the bore sections 22 illustrated in FIGS. 3A and 3B, but also a gap between the inner surface of the tube section 1 and the wall section 21. In other words, it is also acceptable to configure the wall section 21 to be smaller than the flow path 11 so that the gap serving as the bore section 22 is located between the inner surface of the tube section 1 and the wall section 21 when the wall section 21 is arranged in the flow path 11. In a configuration in which the tube section 1 has a circular cylindrical shape, the inside diameter of the tube section 1 (the diameter of the flow path 11) is, for example, approximately 0.5 millimeters. Further, when the cross-sectional shape of the bore section 22 is circular, the diameter of the bore section 22 is, for example, in the range of approximately 0.1 millimeters to 0.3 millimeters. The cross-section of the bore section 22 denotes a cross-section perpendicular to the flow path direction X. In this situation, it is sufficient when the tube section 1 has a shape that makes it possible to guide the aqueous humor through the flow path 11. Accordingly, the shape of the tube section 1 is not limited to the circular cylindrical shape. Further, it is sufficient when the bore section 22 has a shape that allows the aqueous humor to pass therethrough. Accordingly, the cross-sectional shape of the bore section 22 is not limited to being circular.

The flow path 11 has the plurality of throttle sections 2 arranged therein. In the present embodiment, the throttle sections 2 of which the quantity is equal to N are arranged, where N is an integer of 2 or larger. The plurality of throttle sections 2 are arranged in the flow path direction X. Among the plurality of throttle sections 2, a throttle section 2 in an n-th position counted from the throttle section 2 positioned in the most upstream side of the flow path direction X will be referred to as an n-th throttle section Cn, where n is an integer equal to or smaller than N. Accordingly, for example, the throttle section 2 in the first position will be referred to as a first throttle section C1, whereas the throttle section 2 in the second position will be referred to as a second throttle section C2. In FIGS. 1, 4, and 5, four throttle sections 2 are illustrated as examples of the plurality of throttle sections 2 (N = 4).

With respect to each of the plurality of throttle sections 2, the area T of the bore section 22 is smaller than the flow path area U of the flow path 11 (T < U). The area T of the bore section 22 denotes the area of the region enclosed by an edge section 22a of the bore section 22. In other words, the area T of the bore section 22 denotes the area of the region required to close up the bore section 22.

Among the plurality of throttle sections 2, the area T of the bore section 22 of the n-th throttle section Cn will be referred to as an area Tn. Accordingly, for example, the area T of the bore section 22 of the first throttle section C1 in the first position will be referred to as an area T1, whereas the area T of the bore section 22 of the second throttle section C2 in the second position will be referred to as an area T2.

The plurality of throttle sections 2 are configured in such a manner that the more downstream the throttle section 2 is positioned in terms of the flow path direction X, the larger is the area T of the bore section 22. In other words, the area Tn of the bore section 22 of the n-th throttle section Cn is larger than the area T(n-1) of the bore section 22 of the (n-1)-th throttle section C(n-1) (i.e., T(n-1) < Tn).

In the present embodiment, the flow path 11 has an inside diameter of approximately 0.5 millimeters, for example. Further, the bore section 22 of the first throttle section C1 has an inside diameter of approximately 0.1 millimeters, for example. The bore section 22 of the second throttle section C2 has an inside diameter of approximately 0.3 millimeters, for example.

Next, the position of the plate section 3 observed when the implant 10 has been attached to the eye 200 will be described, with reference to FIGS. 4 and 5. FIG. 4 is a schematic cross-sectional view of the implant 10 attached to the eye 200. FIG. 5 is a partial enlarged view of FIG. 4.

As illustrated in FIGS. 4 and 5, the plate section 3 is inserted between a sclera 201 and a Tenon capsule 203. A suture thread is inserted in the suture holes 34 (see FIG. 1) in the plate section 3. Being inserted in the suture holes 34, the suture thread sews together the plate section 3 and the sclera 201. As a result, the plate section 3 is fixed to the sclera 201.

When a certain period of time has elapsed since the plate section 3 is fixed to the sclera 201, biological tissues in the eye grow. Further, the biological tissues in the eye connect the Tenon capsule 203 and the sclera 201 to each other by going through each of the plurality of through holes 36 (see FIG. 1). As a result, the position of the plate section 3 is prevented from being shifted in the eye, so that the position of the plate section 3 in the eye is stabilized.

Due to the presence of the plate section 3, a bleb 204 is formed in the surroundings of the plate section 3 as a reaction of the human body. Due to the sustained presence of the plate section 3, the bleb 204 also keeps being formed. As a result, the plate section 3 floats in the bleb 204. The bleb 204 denotes one or more of: a small follicle, a small vacuole, and a small blister.

Next, the position of the tube section 1 observed when the implant 10 has been attached to the eye 200 will be described, with reference to FIGS. 4 and 5.

As illustrated in FIGS. 4 and 5, the tube section 1 extends from the plate section 3 toward a side of an anterior chamber 202 along the sclera 201. Further, the first end section 1a of the tube section 1 penetrates the sclera 201 so as to be inserted into the anterior chamber 202. When the first end section 1a of the tube section 1 is inserted into the anterior chamber 202, what is penetrated by the first end section 1a is not particularly limited. For example, when being inserted into the anterior chamber 202, the tube section 1 may penetrate a region 205 positioned adjacent to the cornea 211. Further, although the first end section 1a of the tube section 1 is inserted into the anterior chamber 202 in the present embodiment, the present invention is not limited to this example. For instance, the first end section 1a of the tube section 1 may be inserted into the pars plana.

While the implant 10 is attached to the eye 200, the second opening section 13 of the tube section 1 is positioned between the sclera 201 and the Tenon capsule 203.

The tube section 1 has a prescribed part 14. The prescribed part 14 is positioned between the first opening section 12 and the second opening section 13 of the tube section 1. Further, while the implant 10 is attached to the eye 200, the prescribed part 14 is positioned between the sclera 201 of the eye 200 and the conjunctiva 206 of the eye 200. In this situation, the Tenon capsule 203 is positioned between the sclera 201 and the conjunctiva 206. When the prescribed part 14 is positioned between the sclera 201 and the conjunctiva 206 while the implant 10 is attached to the eye 200, the position of the prescribed part 14 relative to the Tenon capsule 203 is not particularly limited. It is sufficient when the prescribed part 14 is positioned between the sclera 201 and the conjunctiva 206. For example, while the implant 10 is attached to the eye 200, the prescribed part 14 may be positioned below the Tenon capsule 203. In that situation, the sclera 201, the prescribed part 14, the Tenon capsule 203, and the conjunctiva 206 are positioned in the order of: the sclera 201, the prescribed part 14, the Tenon capsule 203, and the conjunctiva 206 from the sclera 201 toward the outside of the eye 200. Alternatively, while the implant 10 is attached to the eye 200, the prescribed part 14 may be positioned above the Tenon capsule 203. In that situation, the sclera 201, the prescribed part 14, the Tenon capsule 203, and the conjunctiva 206 are positioned in the order of: the sclera 201, the Tenon capsule 203, the prescribed part 14, and the conjunctiva 206 from the sclera 201 toward the outside of the eye 200. In the present embodiment, the prescribed part 14 is positioned below the Tenon capsule 203.

Further, the flow path 11 includes a prescribed flow path 11a. Within the flow path 11, the prescribed flow path 11a is positioned inside the prescribed part 14. In the present embodiment, the plurality of throttle sections 2 are positioned in the prescribed flow path 11a.

While the implant 10 is attached to the eye 200, the first opening section 12 of the tube section 1 is arranged inside the anterior chamber 202.

Next, functions of the implant 10 will be described, with reference to FIGS. 4 and 5.

As illustrated in FIGS. 4 and 5, the aqueous humor is generated by the ciliary body 207. Further, in a front part of the crystalline lens 208, the aqueous humor supplies nutrition by being circulated to the anterior chamber 202 of the eye 200 through the pupil. When the eye 200 is normal, the aqueous humor is discharged to the outside of the eyeball, through a trabecula 209 and a Schlemm's canal 210. However, for patients having glaucoma, the aqueous humor is prevented from being discharged properly, for example, due to the trabecula 209 being clogged. As a result, the ocular pressure rises beyond a normal range. Further, as a result of the abnormal rise of the ocular pressure, the field of vision and the eyesight may be hindered because damage is caused in the optic nerve by the pressure, for example.

When the implant 10 has been attached to the eye 200, the bleb 204 is formed. Further, the aqueous humor in the anterior chamber 202 flows into the flow path 11 via the first opening section 12. Further, the aqueous humor that has flowed into the flow path 11 flows in the flow path direction X through the flow path 11 and is discharged from the second opening section 13. Further, the aqueous humor that has been discharged from the second opening section 13 is supplied to the inside of the bleb 204 and subsequently permeates to the inside of the biological tissues from the surroundings of the bleb 204. Accordingly, the aqueous humor in the anterior chamber 202 is discharged to the outside of the eye via the flow path 11. As a result, it is possible to prevent an abnormal rise of the ocular pressure.

Next, a relationship between a flow volume Z of the aqueous humor and the quantity of the wall sections 21 arranged in the flow path 11 will be described, with reference to FIGS. 3A, 3B, and 5. The flow volume Z of the aqueous humor denotes, more specifically, the volume of the aqueous humor flowing out of the flow path 11 via the second opening section 13 per unit time period.

As illustrated in FIGS. 3A, 3B, and 5, the plurality of throttle sections 2 are arranged in the flow path 11. Further, with respect to each of the plurality of throttle sections 2, the area T of the bore section 22 is smaller than the flow path area U of the flow path 11 (T < S). Accordingly, every time the aqueous humor flowing in the flow path 11 passes each of the throttle sections 2 (the bore sections 22), a pressure loss is caused in the aqueous humor. As a result, the larger the quantity of the wall sections 21 arranged in the flow path 11 is, the more opportunities there are for the pressure loss to be caused in the aqueous humor, and the smaller is the flow volume Z of the aqueous humor.

Further, it is possible to remove each of the wall sections 21 from the flow path 11. Accordingly, every time one of the wall sections 21 is removed from the flow path 11 so as to decrease the quantity of the wall sections 21 arranged in the flow path 11, the opportunities for the pressure loss to be caused in the aqueous humor are reduced. As a result, every time the quantity of the wall sections 21 arranged in the flow path 11 is decreased, the flow volume Z of the aqueous humor increases.

In this situation, the smaller the area T of the bore section 22 is, the higher the degree of the pressure loss is. Accordingly, the smaller the area T of the bore section 22 is, the smaller the flow volume Z of the aqueous humor is.

As described above with reference to FIGS. 3A, 3B, and 5, the plurality of throttle sections 2 (the wall sections 21 and the bore sections 22) are arranged in the flow path 11. Accordingly, when removing one or more of the plurality of wall sections 21 from the flow path 11, the practitioner is able to predict by approximately how much the ocular pressure will be lowered after the removal of the one or more wall sections 21, based on either or both of: the quantity of the wall sections 21 to be removed; and the area T of the bore section 22 in each of the removed wall sections 21. As a result, it is possible to smoothly perform the procedure of adjusting the ocular pressure after the implant 10 is attached to the eye 200.

Further, the plurality of throttle sections 2 are configured in such a manner that the more downstream the throttle section 2 is positioned in terms of the flow path direction X, the larger the area T of the bore section 22 is. Accordingly, the sizes of the areas T of the plurality of bore sections 22 sequentially increase and change regularly, toward the downstream of the flow path direction X. As a result, it is possible to easily predict by approximately how much the ocular pressure will be lowered after the removal of the one or more wall sections 21. In the present embodiment, the sizes of the areas T of the plurality of bore sections 22 change regularly. However, the size of the area T of each of the plurality of bore sections 22 is not particularly limited, and the sizes of the areas T of the plurality of bore sections 22 do not necessarily have to change regularly. Further, for example, the sizes of the areas T of the plurality of bore sections 22 may be equal to one another.

Next, a configuration used for removing each of the plurality of wall sections 21 arranged in the flow path 11 will be described, with reference to FIG. 5.

As illustrated in FIG. 5, in the present embodiment, each of the plurality of wall sections 21 is removed from the flow path 11 by being irradiated with laser light L. More specifically, as being irradiated with the laser light L, each of the plurality of wall sections 21 absorbs the laser light L. As a result, heat is generated in each of the plurality of wall sections 21 so that each of the plurality of wall sections 21 is destructed by being burnt.

For example, the laser light L may be one of: green laser, yellow laser, and red laser. The laser light L has a relatively long wavelength and a large invasion depth. For example, the laser light L is irradiated by an irradiating section 40 such as a multi-color laser photocoagulator or a green laser photocoagulator. Further, while checking the position of each of the plurality of wall sections 21 by using a microscope, the practitioner manipulates the irradiating section 40 to irradiate the laser light L onto each of the plurality of wall sections 21.

When being irradiated with the laser light L, the tube section 1 passes the laser light L. More specifically, the tube section 1 in the present embodiment passes the laser light L, by having one of a transparent color and a translucent color. In this situation, of the tube section 1, it is sufficient when at least the prescribed part 14 passes the laser light L. More specifically, of the tube section 1, it is sufficient when at least the prescribed part 14 is configured to have one of a transparent color and a translucent color.

When being irradiated with the laser light L, each of the plurality of wall sections 21 absorbs the laser light L. Further, each of the plurality of wall sections 21 has a color that is visible to human beings via the tube section 1. More specifically, each of the plurality of wall sections 21 has a dark color such as black or brown. In the present embodiment, the entire region of each of the plurality of wall sections 21 has a dark color such as black or brown. Each of the plurality of wall sections 21 is of, for example, coloring transparent or translucent silicone with the dark color. In this situation, a human being (the practitioner) visually recognizes each of the plurality of wall sections 21 by using a device such as a microscope.

When the laser light L is irradiated onto the tube section 1 from a lateral side of the tube section 1, the laser light L passes through the tube section 1. More specifically, the laser light L passes through a lateral section of the tube section 1. Further, the laser light L that has passed through the tube section 1 is absorbed by a targeted wall section 21 among the plurality of wall sections 21. As a result, the targeted wall section 21 is removed (destructed by being burned) from the flow path 11. In this situation, the lateral side of the tube section 1 denotes the lateral side of the tube section 1 defined when the flow path direction X is defined as a front-and-back direction.

When the laser light L is irradiated onto each of the plurality of wall sections 21 from the lateral side of the tube section 1 while the implant 10 is attached to the eye 200, the laser light L passes through the conjunctiva 206, the Tenon capsule 203, and the tube section 1. In that situation, the laser light L passes through the conjunctiva 206, the Tenon capsule 203, and the tube section 1 in the order of conjunctiva 206, the Tenon capsule 203, and the tube section 1. Further, the laser light L that has passed through the tube section 1 is absorbed by each of the plurality of wall sections 21. As a result, each of the plurality of wall sections 21 is removed (destructed by being burned) from the flow path 11. In this situation, the conjunctiva 206 and the Tenon capsule 203 are translucent. Accordingly, similarly to the tube section 1 in the present embodiment, the conjunctiva 206 and the Tenon capsule 203 pass the laser light L having a large invasion depth, such as green laser, yellow laser, and red laser.

As described above, it is possible to remove each of the plurality of wall sections 21 from the flow path 11. Accordingly, it is possible to adjust the ocular pressure by removing one or two or more of the plurality of wall sections 21 from the flow path 11, after the implant 10 is attached to the eye 200.

Further, the tube section 1 has one of a transparent color and a translucent color. Also, each of the plurality of wall sections 21 has a color that is visible via the tube section 1. Accordingly, each of the plurality of wall sections 21 can be seen through the tube section 1. As a result, the practitioner is able to recognize the position of each of the plurality of wall sections 21 from the outside 1c of the tube section 1.

Further, within the flow path 11, the plurality of throttle sections 2 (the wall sections 21 and the bore sections 22) are positioned in the prescribed flow path 11a. Accordingly, while the implant 10 is attached to the eye 200, the practitioner is able to visually recognize each of the plurality of wall sections 21 via the conjunctiva 206 and the Tenon capsule 203 of the eye 200 and the prescribed part 14 of the tube section 1, and to thus recognize the position of each of the plurality of wall sections 21. In the present embodiment, the practitioner visually recognizes each of the plurality of wall sections 21 by using a microscope.

Further, while the implant 10 is attached to the eye 200, each of the plurality of wall sections 21 is removed from the flow path 11 by being irradiated with the laser light L passing through the conjunctiva 206 and the Tenon capsule 203 of the eye 200 as well as the prescribed part 14 of the tube section 1. Accordingly, it is possible to remove each of the plurality of wall sections 21 while the implant 10 is attached to the eye 200. As a result, it is possible to smoothly perform the procedure of removing each of the plurality of wall sections 21. When the prescribed part 14 is positioned above the Tenon capsule 203 while the implant 10 is attached to the eye 200, the laser light L passes through the conjunctiva 206 and the tube section 1, but is absorbed by each of the wall sections 21 without passing through the Tenon capsule 203. Accordingly, while the implant 10 is attached to the eye 200, each of the plurality of wall sections 21 is removed from the flow path 11 by being irradiated with the laser light L passing through the conjunctiva 206 of the eye 200 and the prescribed part 14 of the tube section 1.

Next, a procedure (steps S1 through S5) for adjusting the ocular pressure of the eye 200 to which the implant 10 has been attached will be described, with reference to FIG. 6. More specifically, the procedure for adjusting the ocular pressure after glaucoma surgery is performed to attach the implant 10 to the eye 200 will be described. FIG. 6 is a flowchart depicting the procedure of adjusting the ocular pressure of the eye 200 to which the implant 10 has been attached. In this situation, the processes in steps S1 through S5 are performed by using an implant system 100. The implant system 100 includes the implant 10, the irradiating section 40, and a measuring section 50 (see FIG. 5) that measures the ocular pressure.

As illustrated in FIG. 6, in step S1, the ocular pressure of the eye 200 to which the implant 10 has been attached is measured. Specifically, the practitioner measures, by using the measuring section 50 the ocular pressure of the eye 200 to which the implant 10 has been attached. For example, the measuring section 50 is a tonometer. The tonometer may be a Goldmann tonometer, for example.

In step S2, it is determined whether or not the measured ocular pressure is higher than a prescribed upper limit value. The prescribed upper limit value is the upper limit of a range in which the ocular pressure is considered to be normal. For example, the prescribed upper limit value is 21 mmHg, which is the upper limit of ocular pressure that is generally considered normal. Alternatively, the prescribed upper limit value may be smaller than 21 mmHg. For example, the prescribed upper limit value may be 15 mmHg. The reasons is that this makes it possible to adjust the ocular pressure before the ocular pressure reaches 21 mmHg and to ensure that the ocular pressure is normal with higher certainty, while taking into consideration the possibility of the ocular pressure gradually rising after the ocular pressure is measured. When the ocular pressure is higher than the prescribed upper limit value (step S2: Yes), the process proceeds to step S3. On the contrary, when the ocular pressure is equal to or lower than the prescribed upper limit value (step S2: No), the process ends.

In step S3, one of the plurality of wall sections 21 is removed from the flow path 11. More specifically, the wall section 21 of the n-th throttle section Cn is removed from the flow path 11. In that situation, the practitioner manipulates the irradiating section 40 so that the laser light L is irradiated from the irradiating section 40. More specifically, while the implant 10 is attached to the eye 200, the laser light L is irradiated onto the wall section 21 of the n-th throttle section Cn, via the conjunctiva 206 and the Tenon capsule 203 of the eye 200, as well as the tube section 1 (the prescribed part 14). As a result, the wall section 21 of the n-th throttle section Cn is removed from the flow path 11. In the present embodiment, at first, the wall section 21 of the first throttle section C1 is removed from the flow path 11.

In step S4, the ocular pressure of the eye 200 to which the implant 10 is attached is measured.

In step S5, it is determined whether or not the measured ocular pressure is higher than a prescribed target value. When the ocular pressure is higher than the prescribed target value (step S5: Yes), the process proceeds to step S3. In that situation, in step S3, the wall section 21 of the (n+1)-th throttle section C(n+1) is removed from the flow path 11. Further, until the ocular pressure is determined to be equal to or lower than the prescribed target value in step S5, the processes in steps S3 through S5 are repeatedly performed. On the contrary, when the ocular pressure is equal to or lower than the prescribed target value (step S5: No), the process ends. The prescribed target value may be 10 mmHg, for example. It is acceptable when the prescribed target value is 21 mmHg or lower. However, when the possibility of the ocular pressure gradually rising after the ocular pressure adjusting process is finished is taken into consideration, it is desirable to set the prescribed target value at approximately 10 mmHg as in the present embodiment.

As described above with reference to FIG. 6, the processes in steps S3 through S5 are repeatedly performed until the ocular pressure is determined to be equal to or lower than the prescribed target value. Accordingly, by removing the wall sections 21 one by one until the ocular pressure becomes equal to or lower than the prescribed target value, it is possible to gradually increase the flow volume Z of the aqueous humor discharged by the tube section 1 (the second opening section 13) and to thus gradually lower the ocular pressure. As a result, it is possible to perform the procedure of adjusting the ocular pressure in a stable manner, by suppressing drastic change in the ocular pressure.

Certain embodiments of the present invention have thus been described with reference to the drawings (FIGS. 1 to 6). However, the present invention is not limited to the embodiments described above. It is possible to carry out the present invention in various modes (e.g., (1) to (8)) without departing from the gist thereof. Further, it is possible to arrive at various inventions by combining together two or more of the constituent elements disclosed in the above embodiments as appropriate. For example, one or more of the constituent elements described in the embodiments may be omitted. To facilitate the understanding, the drawings are schematically illustrated while a focus is placed on the constituent elements thereof. The quantity and the like of the constituent elements illustrated in the drawings may be different from those in actuality for the sake of convenience in the preparation of the drawings. Further, the constituent elements described in the above embodiments are merely examples, and are not particularly limited. It is possible to apply different variations thereto, without substantially departing from advantageous effects of the present invention.
(1) The implant 10 is attachable, not only to the eye of a human being, but also to the eye of an animal (e.g., a dog) other than human beings. Further, it is possible to apply the method (see steps S1 through S5) for adjusting the ocular pressure of the eye to which the implant 10 has been attached, not only to human beings, but also to other animals besides human beings.
(2) In the present embodiments, the implant 10 includes the plurality of throttle sections 2. However, the present invention is not limited to this example. The implant 10 may include a single throttle section 2. In other words, the implant 10 includes the one or more throttle sections 2. When the implant 10 includes the single throttle section 2, it is possible to keep compact the implant 10 capable of adjusting the ocular pressure. It is also acceptable to prepare a plurality of implants 10 that are different from one another in either or both of: the size of the area T of the bore section 22; and the size of the flow path area U of the flow path 11, so as to select one of the plurality of implants 10 to be attached to the eye of each patient in accordance with the level of seriousness of symptoms of glaucoma (the level of the ocular pressure) of the patient. For example, the implant 10 may be selected in such a manner that the more serious the symptoms of the patient are, the larger the area T of the bore section 22 is and the larger the flow path area U of the flow path 11 is. As a result, it is possible to adjust the ocular pressure effectively, in accordance with the level of seriousness of the symptoms of the patient.
(3) The implant 10 of the present embodiments includes the tube section 1, the plurality of throttle sections 2, and the plate section 3. However, the present invention is not limited to this example. The implant 10 does not necessarily have to include the plate section 3. It is sufficient when the implant 10 includes at least the tube section 1 and one or more throttle sections 2. In other words, it is sufficient when the implant 10 is configured so that the flow path 11 lies inside the tube section 1, while the plurality of throttle sections 2 are arranged in the flow path 11, so that the implant 10 has a function of guiding the aqueous humor generated by the eye 200 to the outside of the eye 200 through the flow path 11. In a configuration in which the implant 10 does not include the plate section 3 but includes the tube section 1 and the one or more throttle sections 2, the implant 10 will be for example in the following states (i), (ii), and (iii) when the implant 10 has been attached to the eye 200: (i) the first end section 1a of the tube section 1 is inserted in the anterior chamber 202 (see FIG. 4); (ii) the prescribed part 14 of the tube section 1 is positioned between the sclera 201 and the conjunctiva 206; and (iii) the second end section 1b of the tube section 1 is directly or indirectly fixed to a rear part of the sclera 201. The rear part of the sclera 201 denotes such a part of the sclera 201 that is positioned behind the eye 200 relative to the conjunctiva 206. As a result, it is possible to simply configure the implant 10, without using the plate section 3.
(4) While the implant 10 is attached to the eye 200, the tube section 1 of the present embodiments is positioned so as to extend from the anterior chamber 202 toward the rear of the eye 200. However, the location in which the tube section 1 can be arranged is not particularly limited. It is sufficient when the tube section 1 is positioned in such a location that allows the aqueous humor to be guided to the outside of the eye 200 through the flow path 11, while the implant 10 is attached to the eye 200.
(5) Variations of the wall sections 21 will be described, with reference to FIG. 7. FIG. 7 is a cross-sectional view illustrating a variation of the wall sections 21. In the embodiments described above, each of the plurality of wall sections 21 is positioned substantially parallel to a perpendicular direction Y (see FIG. 3B). The perpendicular direction Y denotes a direction perpendicular to the flow path direction X. However, the present invention is not limited to this example. As illustrated in FIG. 7, at least one of the plurality of wall sections 21 may be slanted with respect to the perpendicular direction Y The wall section 21 being slanted with respect to the perpendicular direction Y means that a slant angle θ° of a wall surface 21a of the wall section 21 with respect to the perpendicular direction Y is not 0° (θ ≠ 0°). When the wall section 21 is slanted with respect to the perpendicular direction Y, it becomes easier for the practitioner to target the wall section 21 when performing the procedure of removing the wall section 21 by irradiating the laser light L onto the wall section 21 from the lateral side of the tube section 1. As a result, it becomes possible to more smoothly perform the procedure of removing the wall section 21. In this situation, the slant angle θ° is preferably an acute angle. In other words, it is desirable when a lower end section 21b of the wall section 21 is positioned on the upstream side of the flow path direction X with respect to an upper end section 21c of the wall section 21. In a configuration in which the slant angle θ° is an acute angle, when the implant 10 is viewed from the front side (the front face side) of the eye 200 while the implant 10 is attached to the eye 200, a line of sight G toward the implant 10 opposes the wall surface 21a of the wall section 21. As a result, it becomes easier to visually recognize the wall section 21. It is therefore possible to smoothly perform the procedure of removing the wall section 21.
(6) In step S3 illustrated in FIG. 6, the single wall section 21 is removed. However, the present invention is not limited to this example. For instance, when the ocular pressure measured in step S1 is excessively high compared to the prescribed upper limit value, two or more wall sections 21 may be removed in step S3. In other words, according to the level of the ocular pressure measured in step S1, the quantity of the wall sections 21 to be removed in step S3 may be changed. In the present embodiment, the quantity of the wall sections 21 to be removed is determined by the practitioner. In this situation, the quantity of the wall sections 21 to be removed in step S3 may be determined based on empirical rules of the practitioner, for example.

Next, an example of determining the quantity of the wall sections 21 to be removed will be described, with reference to FIG. 8. FIG. 8 is a diagram illustrating change information 80.

The quantity of the wall sections 21 to be removed in step S3 illustrated in FIG. 6 is determined, for example, based on the change information 80. The change information 80 is information representing (A) a wall section 21 to be removed from the flow path 11 among the plurality of wall sections 21, in correspondence with (B) the ocular pressure after the removal of the wall section 21, for each of the wall sections 21. More specifically, the ocular pressure after the removal of a wall section 21 indicates one of the following: a value of the ocular pressure after the removal of the wall section 21; a change amount in the ocular pressure after the removal of the wall section 21; and a change ratio of the ocular pressure after the removal of the wall section 21. In the present embodiments, as illustrated in FIG. 8, the ocular pressure after the removal of a wall section 21 denotes a change ratio of the ocular pressure after the removal of the wall section 21. The change information 80 is obtained by performing experiments in advance. The values of the ocular pressure after the removal of the wall sections 21, the change amounts in the ocular pressure after the removal of the wall sections 21, and the change ratios of the ocular pressure after the removal of the wall sections 21 may each be expressed with a given value or with values having a width defined by an upper limit and a lower limit, such as values in the range from D to E, inclusive, where D and E are real numbers.

A procedure of adjusting the ocular pressure by using the change information 80 will be described. After checking the ocular pressure measured in step S1, the practitioner refers to the change information 80. Further, based on the change information 80, the practitioner selects one or two or more of the wall sections 21 that, when being removed, will make it possible to lower the ocular pressure to a level equal to or lower than the prescribed target value. After that, by manipulating the irradiating section 40, the practitioner irradiates the laser light L onto the wall sections 21 selected by the practitioner. As a result, the wall sections 21 selected by the practitioner will be removed from the flow path 11.

In that situation, the implant system 100 includes either or both of storage that stores therein the change information 80 and a sheet having formed thereon an image representing the change information 80. The storage may be any storage as long as the practitioner is able to access the change information 80 stored therein. The storage may be USB memory, for example. The sheet may be a sheet of paper, for example.

As described above, according to the level of the ocular pressure measured in step S1, the quantity of the wall sections 21 to be removed in step S3 is changed. As a result, it is possible to efficiently perform the procedure of reducing the ocular pressure to the level lower than the prescribed target value.
(7) In the present embodiments, the more downstream the throttle section 2 is positioned in terms of the flow path direction X, the larger the area T of the bore section 22 is. However, the present invention is not limited to this example. The size of the area T of each of the plurality of bore sections 22 is not particularly limited. It is, however, easier to predict the change amounts in the ocular pressure caused by the removal of the wall sections 21, when the sizes of the areas T of the plurality of bore sections 22 are configured so as to regularly change, as described in the present embodiments.
(8) In the present embodiments, when the processes in steps S3 through S5 are repeatedly performed as illustrated in FIG. 6, the wall sections 21 are sequentially removed starting with the wall section 21 of the first throttle section C1. However, the present invention is not limited to this example. When the processes in steps S3 through S5 are repeatedly performed, the order in which the wall sections 21 of the first to the N-th throttle sections (CI to CN) are to be removed are not particularly limited. For instance, one or more wall sections 21 to be removed among the plurality of wall sections 21 may be determined based on the change information 80 described in (5) above.

### [INDUSTRIAL APPLICABILITY]

The present invention is applicable to the field of implants used for treatment of the eyes.

## Claims

1. An implant attachable to an eye, comprising:
a tube section having a flow path for aqueous humor lying therein; and
a throttle section arranged in the flow path, wherein
the tube section has:
a first opening section that allows communication between the flow path and an outside of the tube section; and
a second opening section that allows communication between the flow path and the outside of the tube section, and
the throttle section includes a wall section arranged in the flow path and a bore section penetrating the wall section.

2. The implant of claim 1, wherein
the wall section is removable from the flow path.

3. The implant of claim 2, wherein
the wall section is removed from the flow path by being irradiated with laser light.

4. The implant of claim 3, wherein
the tube section passes the laser light, and
the wall section absorbs the laser light.

5. The implant of claim 4, wherein
the tube section has one of a transparent color and a translucent color, and
the wall section has a color that is visible via the tube section.

6. The implant of claim 5, wherein
the tube section includes a prescribed part that is positioned between a sclera of the eye and a conjunctiva of the eye while the implant is attached to the eye,
the flow path includes a prescribed flow path positioned inside the prescribed part of the tube section, and
the throttle section is positioned in the prescribed flow path.

7. The implant of claim 6, wherein
while the implant is attached to the eye, the wall section is removed from the flow path by being irradiated with one of: the laser light that passes through the conjunctiva of the eye, a Tenon capsule of the eye, and the prescribed part of the tube section; and the laser light that passes through the conjunctiva of the eye and the prescribed part of the tube section.

8. The implant of any one of claims 1 to 7, wherein
the wall section is slanted with respect to a perpendicular direction that is a direction perpendicular to a flow path direction, and
the flow path direction is a direction extending from the first opening section toward the second opening section via the flow path.

9. The implant of any one of claims 1 to 8, comprising
as the throttle section, a plurality of throttle sections arranged in the flow path,
the two or more throttle sections are arranged in the flow path direction,
the flow path direction is a direction extending from the first opening section toward the second opening section via the flow path, and
the plurality of throttle sections are configured in such a manner that the more downstream the throttle section is positioned in terms of the flow path direction, the larger an area of the bore section is.

10. An implant system comprising:
the implant of any one of claims 3 to 7;
an irradiating section configured to irradiate the laser light; and
a measuring section configured to measure ocular pressure.
